# EUROPEAN PATENT SPECIFICATION

(11) **EP 1 996 031 B1**
(45) Date of publication and mention of the grant of the patent: **22.07.2009**
(21) Application number: 07747355.1
(22) Date of filing: 23.03.2007
(51) Int. Cl.: A23L 1/29, A23L 1/30, A23L 1/305, A23L 1/308

(54) **PRETERM FORMULA**
FRÜHGEBURTENFORMEL
FORMULE POUR PRÉMATURÉS

(30) Priority: 23.03.2006 EP 06111638
(43) Date of publication of application: 03.12.2008
(73) Proprietor: N.V. Nutricia, 2712 HM Zoetermeer (NL)
(72) Inventor: GEORGI, Gilda, 63768 Hösback (DE); STAHL, Bernd, 61191 Rosbach-Rodheim (DE); BOEHM, Günther, 61209 Echzell (DE)
(74) Representative: Swinkels, Bart Willem
(86) International application number: PCT/NL2007/050124
(87) International publication number: WO 2007/108690

(56) References cited:
- EP-A- 1 557 096
- US-A- 4 368 204
- US-A1- 2004 071 824
- US-B1- 6 777 391

## Description

### FIELD OF THE INVENTION

The present invention relates to a method for feeding low birthweight infants (LBW infants), very low birthweight infants (VLBW infants), extremely low birthweight infants (ELBW infants) and preterm infants for improving oral tolerance.

### BACKGROUND OF THE INVENTION

Due to an immature digestive tract, babies who are bom prematurely often suffer from digestive tract related problems, such as constipation, swallowing difficulties, gagging, reflux, cramps and digestion problems. Additionally oral nutrition is often not readily tolerated. The consequence is that preterm babies are often kept on parenteral nutrition for a longer period, with a high risk of secondary complications such as infections and gut atrophy. Reducing the time of parenteral nutrition and stimulating oral tolerance is therefore highly desirable.

Many infant formulae for (full) term infants are available on the market. However, these are not optimal for administration to fragile infants, such as preterm infants. The shortcomings of such formulae result in reduced acceptance of the nutrition by the preterm infants.

EP0758846 describes fat compositions primarily for use in nutritionally complete infant formulas in which the constituent palmitic acid oils and oleic acid oils are co-randomized. The invention additionally includes such co-randomized fat compositions with medium-chain triglycerides added, particularly for use in nutritional products for preterm or low birthweight infants.

WO9629881 describes foods for premature babies and infants and dietetic foods as well as a process for their production based on the milk, milk constituents and milk products of mammals. The foods contain some or all of the albumin and beta-lactoglobulin in the form of monomers. The ratio of alpha-lactalbumin to beta lactoglobulin is improved with respect to the milk of ruminants. The foods contain at least the same amount of beta-casein as alpha-s-casein.

### SUMMARY OF THE INVENTION

The present invention relates to feeding low birthweight infants (LBW infants), very low birthweight infants (VLBW infants), extremely low birthweight infants (ELBW infants) and preterm (= premature) infants (hereinafter commonly referred to as "fragile infants") with a formula particularly adapted to stimulate the feed tolerance of the fragile infant. These fragile infants lack a fully matured intestinal tract, and therefore often experience stool problems. Particularly a prolonged transit time causes constipation and ultimately also inhibits the oral tolerance of the food composition. Hence, the present composition can be suitably used to stimulate oral tolerance and/or reduce stool problems and/or reduce infections in fragile infants.

It was found that precipitates of palmitic acid salts are a main contributor to the constipation in preterm infants fed with infant formula. The present composition is particularly suitable to prevent the calcium precipitates from forming. Additionally the present composition stimulates dissolving of the precipitates and preferably stimulates the maturation of the gastrointestinal tract and improves barrier function.

The present invention relates to a nutritional composition suitable for feeding to fragile infants, and wherein several measures are taken to overcome the abovementioned problems. The following measures are taken:
- the palmitic acid content of the nutritional composition is low, resulting in a reduced formation of palmitic calcium soaps, which harden the stool
- the present composition contains prebiotic oligosaccharides, preferably galactose containing indigestible oligosaccharides (GAL oligo); the prebiotic fibers are fermented by the intestinal bacteria, thereby forming short chain fatty acids, lactate, and reducing the intestinal pH; the reduced pH results in a dissociation of said calcium soaps.
- preferably the present composition also contains medium chain fatty acids (MCT; 8:0, 10:0; 12:0); the MCT enhances calcium absorption and/or palmitic acid absorption, further contributing to the abovementioned desirable effects.

In addition to the above effect, the reduced soap formation as a result of the low palmitic acid content and the presence of GAL-oligo also results in an increased bioavailability of calcium. Hence, the present invention also provides a method for stimulating the calcium absorption in a fragile infant.

To supply preterm infants with balanced and optimal utilizable amino acids for improvement of gut maturation and oral tolerance, low threonine protein is used. Suitable protein sources are for example acid whey and/or sweet whey with a reduced glycomacropeptide (GMP) content.

While reducing the palmitic acid content and including in the nutritional composition (non-caloric or low caloric) prebiotic oligosaccharides (preferably GAL-oligo), it is important to maintain an optimal caloric density and osmolarity. Significant deviations from the values of breast milk for these parameters, contribute to an increased incidence of constipation and/or diarrhea and reduced oral tolerance. The present composition preferably has an osmolarity between 250 and 360 mOsm/liter and a caloric density between 0.65 and 0.9 kcal/ml.

However, only dealing with the acute problem of constipation is not optimal as this may only temporarily improve the situation. Therefore it is highly preferred to stimulate gut barrier maturation and include ingredients capable of stimulating the gut maturation. Surprisingly these ingredients ensure a further reduction of time before full enteral feeding can be commenced. The present prebiotic fiber already contributes to improvement of the intestinal barrier function, by improving mucin quality and quantity. The present inventors have also found that low concentrations of long chain polyunsaturated fatty acids (LC PUFA's) effectively stimulate gut barrier integrity and/or reduce gut permeability. Therefore, to further improve intestinal integrity, LC PUFA's are preferably added. The LC PUFA's reduce permeability of the intestinal tract. The combination of GAL-oligo and LC PUFA's synergistically improve barrier integrity.

The advantageous effects of the present invention, such as for example the reduced transit time and reduced constipation, improve oral tolerance of the fragile infant. Furthermore, the improved transit time results in a reduced irritation and prevents infections from occurring, particularly intestinal infections such as colitis (e.g. necrotizing enterocolitis).

### DETAILED DESCRIPTION OF PREFFERED EMBODIMENTS

In one aspect the present invention provides the use of a composition with a threonine content between 100 and 200 mg threonine per 100 kcal comprising: a) between 5 and 15 wt.% palmitic acid based on total fatty acids; and b) prebiotic oligosaccharides capable of reducing the pH in the intestinal tract, for the manufacture of a nutritional composition for reducing the time in which full enteral feeding can be commenced and/or treatment and/or prevention of infection in preterm infants, low birthweight infants (LBW infants), very low birthweight infants (VLBW infants) and/or extremely low birthweight infants (ELBW infants), as further defined in claims 1 and 3.

In a further aspect the present invention provides a composition suitable for administration to preterm infants, low birthweight infants (LBW infants), very low birthweight infants (VLBW infants) and/or extremely low birthweight infants (ELBW infants) with a threonine content between 100 and 200 mg threonine per 100 kcal comprising: a) between 5 and 15 wt.% palmitic acid based on total fatty acids; and b) galactose containing indigestible oligosaccharide, as further defined in claim 4.

### Infants

The present invention relates to feeding an infant selected from the group consisting of low birthweight infants (LBW infants), very low birthweight infant (VLBW infants), extremely low birthweight infants (ELBW) and premature (= preterm) infants, hereinafter commonly referred to as "fragile infants". Low birthweight infants have a weight of less than 2500 grams at birth. Very low birthweight have a weight below 1500 grams at birth. Extremely low birthweight infants have a birthweight below 1000 grams at birth. Preterm (or premature) infants arc born before the end of the 37th week of pregnancy.

### Uses

In one aspect, the present invention provides for the treatment and/or prevention of disorders in fragile infants. In a further aspect the present invention provides a method for feeding fragile infants, comprising administering to said fragile infants the present composition. By administering the present composition, constipation is reduced due to reduced gastrointestinal transit times, softer stool and increased stool frequency. This results in reduced occurrence of gastrointestinal cramps and reduced incidence of infections. Ultimately, the improved intestinal function results in an improved oral tolerance of the feed by the infant and a reduction in time when full enteral feeding can be commenced. This reduces the risk of infections and gut atrophy by parenteral nutrition.

Hence, the present invention provides for:
a) treatment and/or prevention of constipation;
b) reduced gastrointestinal transit times;
c) increased stool frequence;
d) softening stool;
e) treatment and/or prevention of intestinal cramps;
f) treatment and/or prevention of infection;
g) enhancing oral tolerance;
h) stimulating the immunesystem and/or
i) reducing the time on which full enteral feeding can be commenced;
in fragile infants.

In one embodiment the present invention provides for the treatment and/or prevention of an infection selected from the group consisting of necrotizing enterocolitis, intestinal tract infection, respiratory tract infections and urinary tract infections in a fragile infant, in particular by administering to said fragile infant the present composition.

### Palmitic

Palmictic acid (16:0) is an important nutrient in the fragile infants' diet. However, normally the palmitic acid content of nutrition for fragile infants is too high. The high content of palmitic acid causes soap formation, constipation and several other adverse side effects. Therefore the present composition contains 5 to 15 wt.% palmitic acid based on total fatty acid, more preferably 5 to 12.5 wt%. The palmitic acid is preferably included in the composition as triglyceride.

### Oligosaccharide

The composition used in the present method contains a prebiotic oligosaccharide capable of reducing the pH in the intestinal tract. The term oligosaccharides as used in the present invention refers to saccharides which have a degree of polymerization (DP) of saccharide units exceeding 2 and which are not or only partially digested in the intestine by the action of acids or digestive enzymes present in the human upper digestive tract (small intestine and stomach), but which are also fermented by the human intestinal flora. The oligosaccharides as used in the present invention preferably refers to saccharides which have a degree of polymerisation preferably below 60 saccharide units, preferably below 40, even more preferably below 20, most preferably below 10.
Preferably the present oligosaccharide is water-soluble. Water-solublility can be suitably determined according to a method described by L. Prosky et al, J. Assoc. Anal. Chem 71: 1017-1023, 1988. The term "fermentable" as used herein refers to the capability to undergo breakdown by micro-organisms in the lower part of the gastro-intestinal tract (e.g. colon) to smaller molecules, in particular conversion to short chain fatty acids and lactate. In a more preferred embodiment at least 80 wt. % of the present oligosaccharides are prebiotics. "Prebiotics" are defined as non-digestible food ingredients that selectively stimulate the growth and/or activity of one or more bacterial species in the colon and thereby beneficially affect the host (Gibson and Roberfroid, J. Nutr. 125:1401-1412(1995)).

Preferably the present prebiotic oligosaccharide is selected from the group consisting of fructopolysaccharides (e.g. inulin), fructooligosaccharides, indigestible dextrins, galactooligosaccharides (including transgalactooligosaccharides), xylooligosaccharides, arabinooligosaccharides, glucooligosaccharides, mannooligosaccharides, fucooligosaccharides and mixtures thereof. Most preferably the present oligosaccharide is selected from the group consisting of fructooligosaccharides, fructooligosaccharides, and galactooligiosaccharides. Preferably the present prebiotic oligosaccharide is a galactose containing indigestible oligosaccharide.

### Galactose containing oligosaccharide

It was found that galactose containing indigestible oligosaccharides (hereinafter referred to as "GAL-oligo") are particularly effective in reducing the intestinal pH and/or stimulating lactate formation in the intestinal tract (see example 1). This ensures a reduced soap formation and/or stimulates dissolving of precipitated soaps. Hence, the present composition preferably contains galactose containing indigestible oligosaccharide.

The present galactose containing indigestible oligosaccharide (GAL-oligo) preferably contains at least two terminal saccharide units, wherein at least one terminal saccharide unit is selected from the group consisting of glucose and galactose; and at least one terminal saccharide is selected from the group consisting of galactose and fucose. Preferably at least 75 % of the saccharides of the GAL-oligo are β-linked, preferably 100%.

The term "terminal saccharide" refers to a saccharide which is bound to one other saccharide unit (e.g. galactose, glucose, fructose or fucose). The present GAL-oligo preferably contains not more than 4 terminal saccharides, preferably not more than 2.
In a preferred embodiment, the GAL-oligo contains at least one terminal galactose and one selected from at least one terminal glucose and one terminal fucose. Even more preferably, the present GAL-oligo comprises at least one terminal galactose and at least one terminal glucose. Preferably the oligosaccharide consists of 2 terminal saccharide units and 2 to 60 saccharide units in total.

Preferably the GAL-oligo is selected from the group consisting of transgalactooligosaccharides, galactooligosaccharides, lacto-N-tetraose (LNT), lacto-N-neotetraose (neo-LNT), fucosyl-lactose, fucosylated LNT and fucosylated neo-LNT. In a particularly preferred embodiment the present method comprises the administration of transgalactooligosaccharides ([galactose]ₙ-glucose; wherein n is an integer between 1 and 60, i.e. 2, 3, 4, 5, 6, ...., 59 ,60; preferably n is selected from 2, 3, 4, 5, 6, 7, 8, 9 and 10). Transgalactooligosaccharides (TOS) are for example sold under the trademark Vivinal^{™} (Borculo Domo Ingredients, Netherlands). Preferably the saccharides of the transgalactooligosaccharides are β-linked

The present composition preferably comprises 0.1 to 12 grams of the GAL-oligo per 100 gram dry weight of the composition, preferably between 0.5 and 8 grams, more preferably between 1.0 and 7.5 grams. After reconstitution of the powder in liquid and administration of the liquid formula to the infant, these amounts of GAL-oligo provide the desired effects without causing intestinal discomfort.

### Different oligosaccharides

In order to reach an optimal pH lowering effect over the full length of the intestinal tract, the present composition preferably contains two different oligosaccharides. Each oligosaccharide is fermented by a different microorganism in the intestinal flora and/or on a different location in the intestinal tract, resulting in reduced pH due to improved short chain fatty acid (SCFA) formation. Reduced pH and improved SCFA formation reduce soap formation and reduce constipation.

The present invention provides a composition which preferably comprises oligosaccharide A and oligosaccharide B. Oligosaccharide A and oligosaccharide B have different glycosidic linkages, different degree of polymerisation and/or different monosaccharide composition. Preferably oligosaccharide A is a galactose containing indigestible oligosaccharide.

According to a preferred embodiment of the present invention, the percentage of at least one monosaccharide selected from the group consisting of glucose, fructose and galactose in oligosaccharide A is at least 40% higher than the percentage of the same monosaccharide in oligosaccharide B, preferably at least 50%, more preferably at least 75%, even more preferably at least 90%. An increased diversity of monosaccharides stimulate a wider population of intestinal probiotic bacteria, resulting in a improved pH lowering effect and enhanced stimulation of short chain fatty acids. The percentage of a monosaccharide in the saccharide can be simply calculated by dividing the number of the respective monosaccharide unit (e.g. glucose) in the saccharide by the total number of the monosaccharide units in that saccharide.

Preferably oligosaccharide A and B have a degree of polymerisation (DP) between 2 and 200. Preferably at least 80 wt. %, more preferably at least 95 wt. %, most preferably at least 98 wt. % of the cumulative weight of oligosaccharide A and B has a degree of polymerisation (DP) below 100, more preferably below 60, most preferably below 40. The lower DP advantageously reduces viscosity and increases fermentability of the non-digestible saccharides. Preferably at least 50 wt. %, preferably at least 75 wt.% of the cumulative weight of saccharides A and B are non-digestible saccharides with a DP of 2-9. By using a mixture with a high weight percentage of small saccharides the fermentability and stimulation effect on the growth of the lactic acid bacteria and *Bifidobacteria* is increased.

According to a preferred embodiment of the present invention, the DP of oligosaccharide A is at least 5 monosaccharide units lower than the degree of polymerisation of oligosaccharide B, preferably at least 10, even more preferably at least 15. Including a oligosaccharide with an increased degree of polymerisation reduces the osmotic load, which is advantageous for a fragile infant nutrition and improves prebiotic stimulation of the intestinal flora.

Preferably, oligosaccharide A has a DP of 2-9, more preferably 2-8. Preferably oligosaccharide B has DP of 10-100. The saccharides A and B with a different DP may have the same or slightly different monosaccharide composition, preferably different monosaccharide compositions.

In a preferred embodiment of the present invention the percentage of at least one type of glycosidic linkage of saccharide A based on total glycosidic linkages of oligosaccharide A is at least 40% higher the percentage of the same glycosidic linkage in oligosaccharide B, preferably at least 50%, even more preferably at least 75%. The term "glycosidic linkage" as used in the present invention refers to a C-O-C bond formed between the rings of two cyclic monosaccharides by the elimination of water. An increased diversity in glycosidic linkages stimulates a wider range of beneficial bacteria.
Glycosidic linkages differ in that they covalently bind carbon atoms in the monosaccharide units at differently numbered positions, and/or that they form α or β bonds. Examples of different types of glycosidic linkages occurring in non-digestible saccharides are β(1,3), β(1,2), β(1,6) α(1,4), β(2,1), β(2,6), α(1,2), and β(1,4) linkages. Preferably the glycosidic linkages in oligosaccharide A comprises at least 40 % β(1,4) glycosidic linkages, more preferably at least 75%. The glycosidic linkages in oligosaccharide B preferably comprise at least 40 % β(2,1) glycosidic linkages, more preferably at least 75%.

In a preferred embodiment the present composition contains a combination of a galactooligiosaccharide with an average DP between 2 and 10 and an oligosaccharide selected from the group consisting of fructopolysaccharides (e.g. inulin) and fructooligosaccharides, more preferably a combination of galactooligosaccharides and inulin.

### Protein

The present composition contains proteins. The proteins are essential for the growth of the fragile infant. Whey protein is highly suitable as a protein source for fragile infants. However, whey generally has a high threonine content. A high threonine content can result in hyperthreoninemia. Hence, measures are preferably taken to reduce the threonine content of the present composition.
Due to the low inherent threonine content, the present composition preferably contain acid whey or sweet whey from which at least part of the glycomacropeptide (GMP) is removed. The protein component preferably contains non-hydrolysed intact protein, protein hydrolysate and/or amino acids. Preferably the present composition contains non-hydrolysed protein. The present composition contains between 100 and 200 mg threonine per 100 kcal, preferably between 125 and 175 mg threonine per 100 kcal.

### Medium chain fatty acids

Due to the reduced palmitic acid content, the caloric content and fat content of the composition may be significantly reduced. This is however undesirable. Hence, to ensure a sufficient intake of fats and calories, at least part of the fat fraction is generally provided by medium chain triglycerides. The medium chain triglyceride have the advantage over the longer chain fatty acids in that these are more quickly absorbed in the intestinal tract, thereby reducing calcium soap formation and reducing constipation, resulting in an improved oral tolerance. Additionally MCT can be absorbed without lipase being required. Because lipase production in fragile infants is normally limited, the MCT's are particularly suitable for inclusion in nutrition for fragile infants.

Medium chain fatty acids (MCFA) include caprylic acid (C8:0), capric acid (C10:0) and lauric acid (C12:0). These are normally provided in the form of medium chain triglycerides (MCT), wherein MC refers to a chain length comprising C8:0, C10:0 and C 12:0. The present invention preferably contains 2 to 50 wt% medium chain fatty acids based on total weight of fatty acids, preferably between 2 and 25 wt.%, even more preferably between 2 and 10 wt.%. Because lauric acid still has some ability to form calcium soaps, preferably the composition contains less than 25 wt.% lauric acid based on total weight of fatty acids, preferably below 12 wt.%, more preferably below 10 wt.% based on total weight of fatty acids.

### Polyunsaturated fatty acids

The present inventors have also found that eicosapentaenoic acid (EPA, n-3), docosahexaenoic acid (DHA, n-3) and arachidonic acid (AA, n-6) effectively reduce intestinal tight junction permeability. The present inventors found that oral tolerance is partly stimulated by enhancing gut maturation and thus reducing gut permeability. Therefore the present composition preferably comprises at least one long chain polyunsaturated fatty acid selected from the group consisting of EPA, DHA and AA.

Low concentration of LC PUFA's already effectively reduce tight junction permeability Hence, the content of LC PUFA with 20 and 22 carbon atoms in the present composition, preferably does not exceed 10 wt.% of the total fat content, preferably does not exceed 5 wt.%, even more preferably does not exceed 3 wt.% of the total fat content. Preferably the present composition comprises at least 0.1 wt.%, preferably at least 0.25 wt.%, more preferably at least 0.5 wt.%, even more preferably at least 0.75 wt.% LC PUFA with 20 and 22 carbon atoms of the total fat content. The omega-3 LC-PUFA content preferably does not exceed 1 wt.% of the total fat content; the omega-6 LC-PUFA content preferably does not exceed 2 wt.% of the total fat content; the AA (omega-6) content is preferably below 1 wt.% of the total fat content; and/or the weight ratio EPA/DHA is preferably 1 or lower, more preferably below 0.5.
The EPA content preferably does not exceed 5 wt.% of the total fat, more preferably does not exceed 1 wt.%, but is preferably at least 0.05 wt%, more preferably at least 0.1 wt.% of the total fat. The DHA content preferably does not exceed 5 wt.%, more preferably does not exceed 1 wt.%, but is at least 0.1 wt% of the total fat. As AA was found to be particularly effective in reducing tight junction permeability, the present composition comprises relatively high amounts, preferably at least 0.1 wt.%, even more preferably at least 0.25 wt.%, most preferably at least 0.5 wt.% of the total fat.

The present composition preferably comprises between 5 and 75 wt.% polyunsaturated fatty acids based on total fat, preferably between 10 and 50 wt.%.

The LC-PUFA with 20 and 22 carbon atoms may be provided as free fatty acids, in triglyceride form, in phospholipid form, or as a mixture of one of more of the above. The present composition preferably comprises at least one of AA and DHA in phospholipid form.

### Formulation

For providing sufficient nutrition to the infant, the present composition preferably comprises lipid, protein and digestible carbohydrate and is preferably administered in liquid form. The term "liquid food" as used in the present invention includes dry food (e.g. powders) which are accompanied with instructions as to admix said dry food mixture with a suitable liquid (e.g. water).
The present composition is preferably used as an infant formula and preferably comprises 7 to 15 en% protein, 30 to 50 en% carbohydrates and 40 to 60 en% lipid. (en% is short for energy percentage and represents the relative amount each constituent contributes to the total caloric value of the preparation).
A source of digestible carbohydrate may be added to the nutritional formula. Preferably lactose is provided as a digestible carbohydrate source. Lactose ensures an easy digestion in a manner highly resembling the digestion of breast milk. The present composition preferably contains at least 40 wt.% lactose based on total digestible carbohydrate, more preferably at least 60 wt.%, most preferably at least 75 wt.%.

It was found that stool problems may be further reduced by administering the present composition in liquid food with an osmolarity between osmolarity between 200 and 400 mOsm/liter, preferably between 250 and 360 mOsm/l, preferably between 250 and 300 mOsm/l when the composition contains intact (e.g. non-hydrolysed) proteins. The present liquid food preferably has a caloric density between 0.65 and 0.9 kcal/ml.

### EXAMPLES

### Example 1: pH reduction in faces of infants

Infants were fed with formula containing no additive (control); galactooligosaccharide (GOS) (Vivinal-GOS ^{™}, Friesland Foods, The Netherlands); a combination of GOS (Vivinal-GOS™ and inulin (Raftiline ^{™}, Orafti) (GOS/inulin) and breast milk (BM). After 16 weeks the pH of the feaces was determined (seee Table 1).

| **Formula** | **pH of the faeces** |
|---|---|
| Control | 7.1 |
| GOS | 6.5 |
| GOS/inulin | 5.6 |
| BM | 5.7 |

The above indicates the pH lowering effect of the present GAL-oligo and is indicative for the advantageous use of the GAL-oligo in the present low palmitic, low threonine formula. The effective pH lowering effect of the combination of TOS and inulin is indicative for the effective use of two different oligosaccharides in the present composition.

### Example 2: Lactate production of GAL-oligo

The capacity of inulin, galactooligosaccharide (GOS), human milk oligosaccharides (HMO) and a combination of GOS and inulin, to stimulate lactate production in an *in vitro* semi dynamic batch fermentation system using infants feaces was studied.

Fresh faces was obtained from healthy bottle-fed babies and suspended with 500 mg of different prebiotics (see Table 2) As a source of GOS, Vivinal GOS (Friesland Foods, The Netherlands) was used; as a source of inulin RaftilinHP (Orafti) was used; and isolated human milk oligosaccharides (HMO) were used. Samples were taken after 3 hours of incubation. The results are expressed as amounts of lactate formed per g of prebiotic added, and given in Table 2.

**TABLE 2**

| *Prebiotic* | *Lactate* *(mmol*/*g prebiotic)* |
|---|---|
| Blanc | 0 |
| Inulin (500 mg) | 0.003 |
| GOS (500 mg) | 0.180 |
| GOS/inulin (450 + 50 mg) | 0.212 |
| HMO (500 mg) | 0.244 |

From the results, it is clear that the addition of the present GAL-oligo results in high amounts of lactate being produced. This is indicative for the advantageous use of GAL-oligo in the present composition and method.

### Example 3: Preterm formula

Preterm formula containing per 100 ml: 2.5 gram protein (comprising 123.2 mg threonine, which corresponds to 154 mg threonine per 100 kcal for this composition); 7.6 gram carbohydrates (including 6.4 gram lactose); 4.4 gram fat (including 12 wt.% palmitic acid based on total fatty acid, 0.35 wt.% DHA based on total fatty acids, 0.45 wt.% AA based on total fatty acids, 0.1 wt.% EPA based on total fatty acids, 5 wt.% MCT based on total fat, 40 wt.% oleic acid and 13.3 % linoleic acid); 0.8 gram dietary fiber (0.72 gram galactooligosaccharides (Vivinal^{™}, Friesland Foods) and 0.08 gram inulin (Raftilin, Orafti™); caloric density 80 kcal/100 ml; osmolarity 290 mOsm/l.

## Claims

1. Use of a composition with a threonine content between 100 and 200 mg threonine per 100 kcal comprising:
a. between 5 and 15 wt.% palmitic acid based on total fatty acids; and
b. prebiotic oligosaccharides capable of reducing the pH in the intestinal tract,
for the manufacture of a nutritional composition for
(a) enhancing oral tolerance;
(b) reducing gastrointestinal transit times; and/or
(c) reducing the time in which full enteral feeding can be commenced in infants that are bom before the end of the 37^{th} week of pregnancy and/or infants that have a weight of less than 2500 grams at birth and/or infants that have a weight of less than 1500 grams at birth and/or infants that have a weight of less than 1000 grams at birth at birth.

2. The use according to claim 1, wherein the prebiotic oligosaccharide is a galactose containing indigestible oligosaccharide.

3. Use of a composition with a threonine content between 100 and 200 mg threonine per 100 kcal comprising a) between 5 and 15 wt.% palmitic acid based on total fatty acids and b) galactose containing indigestible oligosaccharide for the manufacture of a nutritional composition for feeding to infants that are born before the end of the 37^{th} week of pregnancy and/or infants that have a weight of less than 2500 grams at birth and/or infants that have a weight of less than 1500 grams at birth and/or infants that have a weight of less than 1000 grams at birth.

4. A composition with a threonine content between 100 and 200 mg threonine per 100 kcal and that contains non-hydrolysed protein comprising:
a. between 5 and 15 wt.% palmitic acid based on total fatty acids; and
b. galactose containing indigestible oligosaccharide; and
c. between 2 and 25 wt.% medium chain fatty acids based on total weight of fatty acids.

5. The composition according to claim 4, comprising 7 to 15 en% protein, 30 to 50 en% carbohydrate and 40 to 60 en% lipid.

6. The composition according to claim 4 or 5, with an osmolarity between 250 and 360 mOsm/liter and a caloric density between 0.65 and 0.9 kcal/ml.

7. The composition according to any one of claims 4-6, comprising between 2 and 10 wt.% medium chain fatty acids based on total weight of fatty acids.

8. The composition according to any one of claims 4-7 comprising less than 25 wt% lauric acid based on total weight of fatty acids.

9. The composition according to any one of claims 4-8, comprising a long chain polyunsaturated fatty acid selected from the group consisting of eicosapentaenoic acid (EPA, n-3), docosahexaenoic acid (DHA, n-3) and arachidonic acid (AA n-6).

10. Composition according to any one of claims 4-9 for use as a medicament.

## Patentansprüche

1. Verwendung einer Zusammensetzung mit einem Threoningehalt zwischen 100 und 200 mg Threonin pro 100 kcal, umfassend:
a. zwischen 5 und 15 Gew.% Palmitinsäure basierend auf den Gesamt-fettsäuren; und
b. präbiotische Oligosaccharide, die fähig sind, den pH-Wert im Verdauungstrakt zu senken,
zur Herstellung eines Nährstoffmittels für
a) das Erhöhen der oralen Verträglichkeit;
b) die Verringerung der Magen-Darm-Durchgangszeit; und/oder
c) das Verringern der Zeit, in der eine vollständige enterale Fütterung in Säuglingen begonnen werden kann, die vor dem Ende der 37. Schwangerschaftswoche geboren werden und/oder Säuglingen, die ein Geburtsgewicht von weniger als 2500 g haben und/oder Säuglingen, die ein Geburtsgewicht von weniger als 1500 g haben und/oder Säuglingen, die ein Geburtsgewicht von weniger als 1000 g haben.

2. Verwendung gemäß Anspruch 1, wobei das präbiotische Oligosaccharid eine unverdauliches Oligosaccharid enthaltende Galactose ist.

3. Verwendung einer Zusammensetzung mit einem Threoningehalt zwischen 100 und 200 mg Threonin pro 100 kcal, umfassend a) zwischen 5 und 15 Gew.% Palmitinsäure basierend auf den Gesamtfettsäuren und b) unverdauliches Oligosaccharid enthaltende Galactose zur Herstellung eines Nährstoffmittels zur Fütterung von Säuglingen, die vor dem Ende der 37. Schwangerschaftswoche geboren werden und/oder Säuglingen, die ein Geburtsgewicht von weniger als 2500 g haben und/oder Säuglingen, die ein Geburtsgewicht von weniger als 1500 g haben und/oder Säuglingen, die ein Geburtsgewicht von weniger als 1000 g haben.

4. Zusammensetzung mit einem Threoningehalt zwischen 100 und 200 mg Threonin pro 100 kcal und die nicht-hydrolysiertes Protein enthält, umfassend:
a. zwischen 5 und 15 Gew.% Palmitinsäure basierend auf den Gesamt-fettsäuren; und
b. unverdauliches Oligosaccharid enthaltende Galactose; und
c. zwischen 2 und 25 Gew.% Fettsäuren von mittlerer Kettenlänge basierend auf dem Gesamtgewicht der Fettsäuren.

5. Zusammensetzung gemäß Anspruch 4, umfassend 7 bis 15 Energie% Protein, 30 bis 50 Energie% Kohlenhydrate und 40 bis 60 Energie% Lipid.

6. Zusammensetzung gemäß Anspruch 4 oder 5, mit einer Osmolarität zwischen 250 und 360 mOsm/Liter und einer kalorischen Dichte zwischen 0,65 und 0,9 kcal/ml.

7. Zusammensetzung gemäß einem der Ansprüche 4 bis 6, umfassend zwischen 2 und 10 Gew.% Fettsäuren von mittlerer Kettenlänge basierend auf dem Gesamtgewicht der Fettsäuren.

8. Zusammensetzung gemäß einem der Ansprüche 4 bis 7, umfassend weniger als 25 Gew.% Laurinsäure basierend auf dem Gesamtgewicht der Fettsäuren.

9. Zusammensetzung gemäß einem der Ansprüche 4 bis 8, umfassend eine langkettige, mehrfach ungesättigte Fettsäure ausgewählt aus der Gruppe bestehend aus Eicosapentaensäure (EPA, n-3), Docosahexaensäure (DHA, n-3) und Arachidonsäure (AA, n-6).

10. Zusammensetzung gemäß einem der Ansprüche 4 bis 9 zur Verwendung als ein Medikament.

## Revendications

1. Utilisation d'une composition ayant une teneur en thréonine comprise entre 100 et 200 mg de thréonine par 100 kcal, comprenant :
a. entre 5 et 15 % en poids d'acide palmitique sur la base du total des acides gras ; et
b. des oligosaccharides prébiotiques capables de réduire le pH dans le tube digestif, pour la fabrication d'une composition nutritionnelle permettant
(a) d'améliorer la tolérance orale ;
(b) de réduire les temps de transit gastro-intestinal ; et/ou
(c) de réduire le moment où l'alimentation entérale totale peut être commencée chez les bébés qui sont nés avant la fin de la 37^{ème} semaine de grossesse et/ou les bébés qui ont un poids inférieur à 2500 grammes à la naissance et/ou les bébés qui ont un poids inférieur à 1500 grammes à la naissance et/ou les bébés qui ont un poids inférieur à 1000 grammes à la naissance.

2. Utilisation selon la revendication 1, dans laquelle l'oligosaccharide prébiotique est un galactose contenant un oligosaccharide indigestible.

3. Utilisation d'une composition ayant une teneur en thréonine comprise entre 100 et 200 mg de thréonine par 100 kcal comprenant a) entre 5 et 15 % en poids d'acide palmitique sur la base du total des acides gras et b) la galactose contenant un oligosaccharide indigestible pour la fabrication d'une composition nutritionnelle pour alimenter les bébés qui sont nés avant la fin de la 37^{ème} semaine de grossesse et/ou les bébés qui ont un poids inférieur à 2500 grammes à la naissance et/ou les bébés qui ont un poids inférieur à 1500 grammes à la naissance et/ou les bébés qui ont un poids inférieur à 1000 grammes à la naissance.

4. Composition ayant une teneur en thréonine entre 100 et 200 mg de thréonine par 100 kcal et qui contient une protéine non-hydrolysée comprenant :
a. entre 5 et 15 % en poids d'acide palmitique sur la base du total des acides gras ; et
b. l'oligosaccharide indigestible contenant du galactose ; et
c. entre 2 et 25 % en poids des acides gras à chaîne moyenne sur la base du poids total des acides gras.

5. Composition selon la revendication 4, comprenant 7 à 15 en% de protéine, 30 à 50 en% de glucides et 40 à 60 en% de lipide.

6. Composition selon les revendications 4 ou 5, ayant une osmolarité comprise entre 250 et 360 mOsm/litre et une densité calorique comprise entre 0,65 et 0,9 kcal/ml.

7. Composition selon l'une quelconque des revendications 4-6, comprenant entre 2 et 10 % en poids d'acides gras à chaîne moyenne sur la base du poids total d'acides gras.

8. Composition selon l'une quelconque des revendications 4-7, comprenant moins de 25 % en poids d'acide laurique, sur la base du poids total des acides gras.

9. Composition selon l'une quelconque des revendications 4-8, comprenant un acide gras polyinsaturé à chaîne longue, choisi dans le groupe constitué d'acide eicosapentaénoïque (EPA, n-3), d'acide docosahexaénoïque (DHA, n-3) et d'acide arachidonique (AA, n-6).

10. Composition selon l'une quelconque des revendications 4-9 à utiliser comme médicament.
